# EUROPEAN PATENT APPLICATION

(11) **EP 4 268 825 A1**
(43) Date of publication of application: **01.11.2023**
(21) Application number: 21910981.6
(22) Date of filing: 23.12.2021
(51) Int. Cl.: A61K 31/55, A61K 9/06, A61K 9/10, A61K 9/107, A61K 47/02, A61K 47/06, A61K 47/10, A61K 47/12, A61K 47/14, A61K 47/18, A61K 47/20, A61K 47/22, A61K 47/44, A61P 27/02, A61P 27/14, A61P 39/06

(54) **PHARMACEUTICAL COMPOSITION FOR TRANSDERMAL ADMINISTRATION CONTAINING EPINASTINE OR SALT THEREOF AND CONTAINING SULFUR-BASED ANTIOXIDANT**

(30) Priority: 24.12.2020 JP 2020215228
(71) Applicant: Santen Pharmaceutical Co., Ltd., Kita-ku Osaka-shi Osaka 530-8552 (JP)
(72) Inventor: FUJISAWA, Toyomi, Ikoma-shi, Nara 630-0101 (JP); IKEDA, Hiroyuki, Kasukabe-shi, Saitama 344-0057 (JP)
(74) Representative: Müller-Boré & Partner Patentanwälte PartG mbB
(86) International application number: PCT/JP2021/047943
(87) International publication number: WO 2022/138842

(57) **Abstract**

The present invention provides a pharmaceutical composition for transdermal administration comprising epinastine or a salt thereof as an active ingredient which can maintain the effective concentration of epinastine or a salt thereof in an ocular tissue and stabilize epinastine or a salt thereof in the pharmaceutical composition for a long time.

## Description

### TECHNICAL FIELD

The present invention relates to a pharmaceutical composition for transdermal administration comprising epinastine or a salt thereof and a sulfur-based antioxidant (hereinafter also referred to as "the pharmaceutical composition of the present invention").

### BACKGROUND ART

Pharmaceutical products must be strictly controlled to ensure the efficacy and safety thereof. For example, the "guideline on Impurities in New Drug Products" defines the quantitation limit value (threshold) for degradation products observed during manufacture or stability studies of a new drug product (degradation products of a drug substance or reaction products of a drug substance with an excipient or immediate container closure system) to ensure the pharmaceutical quality. Also, it is defined that the structures of the degradation products must be determined and the safety of the degradation products must be also confirmed, if the value is exceeded. Hence, in the development of pharmaceutical products, it is required to control the formulation design and manufacturing process so that degradation products are not generated at a level greater than the threshold during the manufacture or storage of pharmaceutical products.

For example, Alesion^{®} 0.05% ophthalmic solution comprising epinastine hydrochloride as an active ingredient, which is used as an agent for treating allergic conjunctivitis, is known as pharmaceutical products comprising epinastine or a salt thereof. Such ophthalmic solution is usually used in a dose and usage of 1 drop per time, four times daily (Non-Patent Document 1). In an ophthalmic solution, it is desirable to reduce the administration frequency thereof in terms of medication adherence. On the other hand, the reduced administration frequency thereof may reduce the drug efficacy of an active ingredient because the effective concentration of the active ingredient in an ocular tissue is not maintained. Hence, it is required to maintain the effective concentration thereof in an ocular tissue to produce the drug efficacy. As a method of maintaining the effective concentration of an active ingredient in an ocular tissue, there is a method of increasing the concentration of an active ingredient in formulation. On the other hand, an increase in the concentration of the active ingredient in formulation may enhance the risk of developing side effects. Hence, in order to maintain the effective concentration of an active ingredient in an ocular tissue, the attempts to select an administration method other than ophthalmic administration have recently been done.

For example, Patent Document 1 discloses a transdermal absorption formulation for treatment of an ophthalmic disease having a structure that a plaster layer containing an agent for treating an ophthalmic disease is provided on a support for adhering the transdermal absorption formulation to a skin surface including a front surface of an eyelid to percutaneously transfer the agent for treating the ophthalmic disease in the plaster layer to an ophthalmic topical tissue by percutaneous permeation substantially without being administered through a systemic blood flow. Also, an ophthalmic transdermal absorption formulation has been reported as a formulation comprising epinastine or a salt thereof other than an ophthalmic solution (Patent Document 2).

### PRIOR ART DOCUMENTS

### PATENT DOCUMENTS

Patent Document 1: WO 2004/064817
Patent Document 2: WO 2007/007851

### NON-PATENT DOCUMENTS

Non-Patent Document 1: Alesion^{®} 0.05% ophthalmic solution, Package insert

### SUMMARY OF INVENTION

### (PROBLEM TO BE SOLVED BY THE INVENTION)

As for the pharmaceutical products comprising epinastine or a salt thereof, it has been known that the stability of epinastine or a salt thereof in aqueous ophthalmic solution is good. On the other hand, it has not been known that it is difficult to stabilize epinastine or a salt thereof in other dosage forms, for example, transdermal absorption formulation for a long time. Also, a method of stabilizing epinastine or a salt thereof in transdermal absorption formulation has not been reported.

Hence, an object of the present invention is to provide a novel pharmaceutical composition comprising epinastine or a salt thereof as an active ingredient which can maintain the effective concentration of epinastine or a salt thereof in an ocular tissue and stabilize epinastine or a salt thereof in the pharmaceutical composition for a long time.

### (MEANS FOR SOLVING THE PROBLEMS)

The present inventors have intensively studied to develop a novel pharmaceutical composition comprising epinastine or a salt thereof, and then surprisingly have found that when a pharmaceutical composition comprising epinastine or a salt thereof is prepared as a transdermal absorption formulation, it is difficult to stabilize epinastine or a salt thereof in the transdermal absorption formulation for a long time because related substances of epinastine or a salt thereof are generated as impurities, contrary to the good stability of epinastine or a salt thereof in aqueous ophthalmic solution. The present inventors have further intensively studied, and then have found that the addition of an antioxidant, specifically a sulfur-based antioxidant into the pharmaceutical composition for transdermal administration comprising epinastine or a salt thereof can inhibit the generation and increase of the related substances under long-term storage to stabilize epinastine or a salt thereof in the pharmaceutical composition for a long time. Based upon the new findings, the present invention has been completed.

Specifically, the present invention provides the following embodiments.
(1) A pharmaceutical composition for transdermal administration comprising epinastine or a salt thereof and a sulfur-based antioxidant.
(2) The pharmaceutical composition according to the above item (1), wherein the sulfur-based antioxidant is one or more selected from the group consisting of cysteine, N-acetylcysteine, methionine, glutathione, 2-mercaptobenzimidazole, sodium thiomalate, thioglycerol, sodium thioglycolate, sodium thiosulfate, sodium hydrogen sulfite and sodium sulfite.
(3) The pharmaceutical composition according to the above item (2), wherein the sulfur-based antioxidant is 2-mercaptobenzimidazole.
(4) The pharmaceutical composition according to any one of the above items (1) to (3), wherein the concentration of the epinastine or a salt thereof is 0.01 to 5% (w/w).
(5) The pharmaceutical composition according to any one of the above items (1) to (4), wherein the pharmaceutical composition is ointment formulation, cream formulation or gel formulation.
(6) The pharmaceutical composition according to any one of the above items (1) to (5), wherein the pharmaceutical composition is cream formulation.
(7) The pharmaceutical composition according to any one of the above items (1) to (6), wherein the pharmaceutical composition is water-in-oil emulsion.
(8) The pharmaceutical composition according to any one of the above items (1) to (7) for ophthalmic use.
(9) The pharmaceutical composition according to any one of the above items (1) to (8), wherein the transdermal administration is the administration to eyelid skin.
(10) The pharmaceutical composition according to any one of the above items (1) to (9) for treating allergic conjunctivitis.
(11) The pharmaceutical composition according to any one of the above items (1) to (10) for use in the administration to a patient once daily.
(12) A pharmaceutical composition for transdermal administration comprising epinastine or a salt thereof and a sulfur-based antioxidant, wherein the pharmaceutical composition further comprises one or more oil ingredients selected from the group consisting of a hydrocarbon, a wax, an oil and fat, an aliphatic carboxylic acid or a salt thereof, a fatty acid ester and a higher alcohol.
(13) The pharmaceutical composition according to the above item (12) which further comprises a solvent and/or a dispersion medium.
(14) The pharmaceutical composition according to the above item (12) or (13), wherein the pharmaceutical composition is free of a paraben.
   In addition, the present invention provides the following embodiments.
(15) The pharmaceutical composition according to any one of the above items (1) to (14), wherein the epinastine or a salt thereof is epinastine.
(16) The pharmaceutical composition according to any one of the above items (1) to (14), wherein the concentration of the sulfur-based antioxidant is 0.01 to 2% (w/w).
(17) The pharmaceutical composition according to any one of the above items (1) to (13), wherein the pharmaceutical composition is free of a preservative.
(18) A method of inhibiting the generation and increase of a related substance of epinastine or a salt thereof, by adding a sulfur-based antioxidant into a pharmaceutical composition for transdermal administration comprising epinastine or a salt thereof.
(19) A method of improving the stability of epinastine or a salt thereof in pharmaceutical composition, by adding a sulfur-based antioxidant into a pharmaceutical composition for transdermal administration comprising epinastine or a salt thereof.
(20) A method of treating allergic conjunctivitis, which comprises administering a therapeutically effective amount of the pharmaceutical composition according to any one of the above items (1) to (17) in a patient in need thereof.
(21) Use of the pharmaceutical composition according to any one of the above items (1) to (17) in the manufacture of a medicament for treating allergic conjunctivitis.
(22) A pharmaceutical composition for transdermal administration comprising epinastine or a salt thereof and an antioxidant.

Each feature of the above (1) to (22) may be optionally selected and combined two or more.

### (EFFECTS OF THE INVENTION)

According to the present invention, a pharmaceutical composition for transdermal administration comprising epinastine or a salt thereof as an active ingredient, which can maintain the effective concentration of epinastine or a salt thereof in an ocular tissue and stabilize epinastine or a salt thereof in the pharmaceutical composition for a long time, can be provided. In addition, the pharmaceutical composition has the sufficient safety for using as a pharmaceutical product.

### DETAILED DESCRIPTION OF THE PREFERRED EMBODIMENTS

Hereinafter, the present invention is explained in detail.

"Epinastine" as used herein is a compound represented by chemical name: (±)-3-Amino-9,13b-dihydro-1H-dibenz[c,f]imidazo[1,5-a]azepine, and also the following formula:

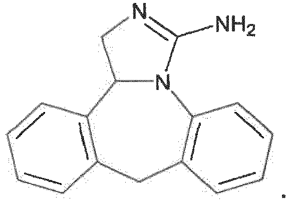

Epinastine in the pharmaceutical composition of the present invention may be in the form of racemate or optical isomer.

Epinastine in the pharmaceutical composition of the present invention may be in a salt form, and it is not particularly limited as long as the salt is a pharmaceutically acceptable salt. Examples thereof include salts with an inorganic acid, an organic acid, and others.

Examples of the salts with an inorganic acid include salts with hydrochloric acid, hydrobromic acid, hydroiodic acid, nitric acid, sulfuric acid, phosphoric acid, and others.

Examples of the salts with an organic acid include salts with acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid, and others.

Particularly preferably, the salt of epinastine is monohydrochloride (epinastine hydrochloride).

Epinastine or a salt thereof in the pharmaceutical composition of the present invention may be in the form of a hydrate or a solvate.

When the pharmaceutical composition of the present invention is prepared, epinastine in the pharmaceutical composition may be in a salt form, but is more preferably in free form to enhance the transdermal absorption of epinastine more efficiently. In the preparation process of the pharmaceutical composition of the present invention, epinastine in free form may be used to prepare the pharmaceutical composition. Also, epinastine in free form may be generated in the pharmaceutical composition by the desalination of a salt of epinastine (e.g., epinastine hydrochloride) by the reaction of the salt of epinastine and an appropriate amount of a base (e.g., sodium hydroxide) in the preparation process.

The amount of epinastine or a salt thereof in the pharmaceutical composition of the present invention is not particularly limited as long as it is a sufficient amount to produce the therapeutic effect desired as a medicament. The amount thereof is preferably 0.01 to 5% (w/w), more preferably 0.03 to 3% (w/w), furthermore preferably 0.05 to 1% (w/w), even furthermore preferably 0.05 to 0.5% (w/w), particularly preferably 0.1 to 1% (w/w). Also, the amount thereof may be preferably 0.01% (w/w), 0.03% (w/w), 0.05% (w/w), 0.1% (w/w), 0.2% (w/w), 0.25% (w/w), 0.3% (w/w), 0.4% (w/w), 0.5% (w/w), 0.6% (w/w), 0.7% (w/w), 0.75% (w/w), 0.8% (w/w), 0.9% (w/w), 1% (w/w), 1.5% (w/w), 2% (w/w), 3% (w/w) or 5% (w/w).

The term "% (w/w)" as used herein means the mass (g) of an object ingredient in 100 g of the pharmaceutical composition of the present invention. When the pharmaceutical composition of the present invention comprises a salt of epinastine, the value means the amount of the salt of epinastine. Also, when the pharmaceutical composition of the present invention comprises epinastine or a salt thereof in the form of a hydrate or a solvate, the value means the amount of the hydrate or the solvate of epinastine or a salt thereof. Hereinafter, the same shall apply to the followings unless otherwise specified.

The "antioxidant" as used herein is not limited as long as it is an antioxidant available as normal pharmaceutical additives. Examples thereof include ascorbic acid, tocopherol or a derivative thereof, propyl gallate, dibutylhydroxytoluene (hereinafter also referred to as "BHT"), cysteine, N-acetylcysteine, methionine, glutathione, 2-mercaptobenzimidazole (hereinafter also referred to as "MBI"), sodium thiomalate, thioglycerol, sodium thioglycolate, sodium thiosulfate, sodium hydrogen sulfite, sodium sulfite, and others. The antioxidant of the present invention is preferably a sulfur-based antioxidant. The antioxidant in the pharmaceutical composition of the present invention may be in the form of a salt, a hydrate or a solvate, or a hydrate or a solvate of the salt. Also, when the antioxidant has one or more chiral centers, it may be used as racemate or optical isomer.

The "sulfur-based antioxidant" as used herein refers to an antioxidant having sulfur atom(s) in the molecule and is also called as a sulfur-based antioxidant agent. Examples thereof include cysteine, N-acetylcysteine, methionine, glutathione, 2-mercaptobenzimidazole (MBI), sodium thiomalate, thioglycerol, sodium thioglycolate, sodium thiosulfate, sodium hydrogen sulfite, sodium sulfite, and others. They may be in the form of a hydrate or a solvate thereof. The sulfur-based antioxidant is particularly preferably 2-mercaptobenzimidazole.

In the pharmaceutical composition of the present invention, the sulfur-based antioxidant may be used alone and two or more sulfur-based antioxidants may be used in combination.

The pharmaceutical composition of the present invention may comprise the sulfur-based antioxidant and any other type of antioxidant. Examples of the antioxidant other than the sulfur-based antioxidant include ascorbic acid, tocopherol or a derivative thereof, propyl gallate, dibutylhydroxytoluene (BHT), and others.

When the pharmaceutical composition of the present invention comprises an antioxidant in addition to the sulfur-based antioxidant, such antioxidant may be used alone and two or more antioxidants may be used in combination.

The amount of the sulfur-based antioxidant as well as the amount of the sulfur-based antioxidant and any other type of antioxidant in the pharmaceutical composition of the present invention may be suitably varied depending on, for example, the number and type of the antioxidants. The amount thereof is, for example, 0.01 to 2% (w/w), preferably 0.03 to 1.5% (w/w), more preferably 0.05 to 1% (w/w), furthermore preferably 0.05 to 0.5% (w/w).

When the pharmaceutical composition of the present invention comprises 2-mercaptobenzimidazole as a sulfur-based antioxidant, the amount of 2-mercaptobenzimidazole is preferably 0.5% (w/w) or less because the compound is insoluble in water. Also, the amount of 2-mercaptobenzimidazole for producing the antioxidant effect is preferably 0.01% (w/w) or more. The amount of 2-mercaptobenzimidazole added in the pharmaceutical composition of the present invention is preferably 0.01 to 0.5% (w/w), more preferably 0.03 to 0.3% (w/w), furthermore preferably 0.05 to 0.3% (w/w). For example, the amount thereof may be 0.05% (w/w), 0.1% (w/w), 0.15% (w/w), 0.2% (w/w), 0.25% (w/w) or 0.3% (w/w).

The pharmaceutical composition of the present invention may be applied topically. For example, the pharmaceutical composition may be prepared as ophthalmic, otorhinological or dermatological pharmaceutical composition. The pharmaceutical composition of the present invention is particularly preferably an ophthalmic transdermal absorption formulation.

The dosage form of the pharmaceutical composition of the present invention is not particularly limited as long as it can be used as a pharmaceutical product. Examples thereof include ointment formulation, cream formulation, gel formulation, patch formulation (tape formulation, cataplasm), spray formulation (e.g., pump spray formulation, external aerosol formulation), external solution (e.g., lotion formulation, liniment formulation), external solid formulation (e.g., external powder), and others. The dosage form of the pharmaceutical composition of the present invention is preferably ointment formulation, cream formulation, gel formulation, patch formulation, spray formulation and external solution, more preferably ointment formulation, cream formulation and gel formulation, particularly preferably cream formulation.

They can be prepared according to a method commonly used in the art.

When the pharmaceutical composition of the present invention is used as an ophthalmic transdermal absorption formulation, the pharmaceutical composition is preferably administered to the area near the eye. The term "area near the eye" refers to the eyelids (e.g., the upper eyelid, the lower eyelid) and the area near the eyelid or the periorbital area. Also, the term includes the skin of each eyelid and the skin of the area near the eyelid or the periorbital area skin. The administration to the area near the eye includes, for example, the application or adhesion to the skin of the upper eyelid, the lower eyelid or both eyelids and the area near the eyelid, or the application or adhesion to the periorbital area skin. The edge of eyelid may be included in the area near the eye. On the other hand, when a portion of the pharmaceutical composition administered to the edge of eyelid comes in contact with the sensitive ocular surface with a blink of eyes, the possibility of producing ocular irritation is increased. Hence, it is more preferable not to administer the pharmaceutical composition to the edge of eyelid.

The pharmaceutical composition of the present invention may comprise an additive for pharmaceutical products as appropriate. For example, an additive such as pH adjuster, buffering agent, tonicity agent, viscosity agent, stabilizing agent, preservative, surfactant, cooling agent and oil ingredient may be added. They may be used alone, respectively, and two or more of them may be used in combination. Also, they may be added in an appropriate amount.

In the pharmaceutical composition of the present invention, a pH adjuster available as pharmaceutical additives may be added at appropriate. Examples of the pH adjuster include an acid or a base. Examples of the acid include hydrochloric acid, phosphoric acid, acetic acid, citric acid, a salt thereof, and others. Examples of the base include sodium hydroxide, potassium hydroxide, sodium carbonate, sodium hydrogen carbonate, and others. Also, they may be in form of a hydrate or a solvate.

The pH of the pharmaceutical composition of the present invention is not limited as long as it is within a range acceptable for pharmaceutical products. The pH thereof is within a range of, for example, 4.0 to 8.5 or 4.0 to 8.0, preferably 6.0 to 8.0, more preferably 6.5 to 7.5. The pH thereof is particularly preferably 6.7 to 7.3. For example, the pH thereof may be 6.7, 6.8, 6.9, 7.0, 7.1, 7.2 or 7.3.

In the pharmaceutical composition of the present invention, a buffering agent available as pharmaceutical additives may be added at appropriate. Examples of the buffering agent include phosphoric acid or a salt thereof, boric acid, borax, trometamol, an organic acid or a salt thereof, and others. They may be in the form of a hydrate or a solvate.

Examples of the phosphoric acid or a salt thereof include phosphoric acid, sodium phosphate, sodium dihydrogen phosphate, disodium hydrogen phosphate, potassium phosphate, potassium dihydrogen phosphate, dipotassium hydrogen phosphate, and others.

Examples of the organic acid include citric acid, acetic acid, ε-aminocaproic acid, gluconic acid, fumaric acid, lactic acid, ascorbic acid, succinic acid, maleic acid, malic acid, amino acid, and others. Examples of the salt thereof include sodium salt, potassium salt, and others.

The amount of the buffering agent in the pharmaceutical composition of the present invention may be suitably varied depending on, for example, the type of the buffering agent. The amount thereof is, for example, 0.01 to 5% (w/w), but this is not applied if the buffering agent produces any effect other than the effect as buffering agent. Also, when the pharmaceutical composition of the present invention comprises a buffering agent, the buffering agent may be used alone and two or more buffering agents may be used in combination.

Phosphoric acid or a salt thereof and the organic acid or a salt thereof in the pharmaceutical composition of the present invention may act as pH adjuster and buffering agent.

In the pharmaceutical composition of the present invention, a tonicity agent available as pharmaceutical additives may be added as appropriate. Examples of the tonicity agent include an ionic tonicity agent, a non-ionic tonicity agent, and others.

Examples of the ionic tonicity agent include sodium chloride, potassium chloride, calcium chloride, magnesium chloride, and others. They may be in the form of a hydrate or a solvate.

Examples of the non-ionic tonicity agent include glycerin (concentrated glycerin), propylene glycol, 1,3-butylene glycol, polyethylene glycol, sorbitol, mannitol, trehalose, maltose, sucrose, xylitol, and others. They may be in the form of a hydrate or a solvate.

The amount of the tonicity agent in the pharmaceutical composition of the present invention may be suitably varied depending on, for example, the type of the tonicity agent. The amount thereof is, for example, 0.01 to 5% (w/w), preferably 0.05 to 3% (w/w), more preferably 0.1 to 2% (w/w), furthermore preferably 0.1 to 1% (w/w), but this is not applied if the tonicity agent produces any effect other than the effect as tonicity agent. Also, when the pharmaceutical composition of the present invention comprises a tonicity agent, the tonicity agent may be used alone and two or more tonicity agents may be used in combination.

In the pharmaceutical composition of the present invention, a viscosity agent available as pharmaceutical additives may be added as appropriate. Examples of the viscosity agent include methyl cellulose, ethyl cellulose, hydroxymethylcellulose, hydroxyethyl cellulose, hydroxypropyl cellulose, hydroxyethylmethyl cellulose, hydroxypropylmethyl cellulose, carboxymethyl cellulose, sodium carboxymethyl cellulose, hydroxypropyl methylcellulose acetate succinate, hydroxypropylmethyl cellulose phthalate, carboxymethylethyl cellulose, cellulose acetate phthalate, polyvinylpyrrolidone, polyvinyl alcohol, carboxyvinyl polymer, polyethylene glycol, sodium hyaluronate, and others. They may be in the form of a hydrate or a solvate.

The amount of the viscosity agent in the pharmaceutical composition of the present invention may be suitably varied depending on, for example, the type of the viscosity agent. The amount thereof is, for example, 0.01 to 20% (w/w), preferably 0.1 to 10% (w/w), more preferably 1 to 10% (w/w), but this is not applied if the viscosity agent produces any effect other than the effect as viscosity agent. Also, when the dosage form of the pharmaceutical composition of the present invention is gel formulation, the amount thereof may be, for example, 20% (w/w) or more. In addition, when the pharmaceutical composition of the present invention comprises a viscosity agent, the viscosity agent may be used alone and two or more viscosity agents may be used in combination.

In the pharmaceutical composition of the present invention, a stabilizing agent available as pharmaceutical additives may be added at appropriate. Examples of the stabilizing agent include edetic acid or a salt thereof, cyclodextrin, and others. They may be in the form of a hydrate or a solvate.

Examples of the edetic acid or a salt thereof include edetic acid, disodium edetate (sodium edetate), tetrasodium edetate, and others.

The amount of the stabilizing agent in the pharmaceutical composition of the present invention may be suitably varied depending on, for example, the type of the stabilizing agent. The amount thereof is, for example, 0.01 to 5% (w/w), preferably 0.01 to 3% (w/w), more preferably 0.01 to 1% (w/w), but this is not applied if the stabilizing agent produces any effect other than the effect as stabilizing agent. In addition, when the pharmaceutical composition of the present invention comprises a stabilizing agent, the stabilizing agent may be used alone and two or more stabilizing agents may be used in combination.

In the pharmaceutical composition of the present invention, a preservative available as pharmaceutical additives may be added at appropriate. Examples of the preservative include benzalkonium chloride, benzalkonium bromide, benzethonium chloride, chlorhexidine gluconate, chlorhexidine hydrochloride, paraben, sodium chlorite, phenoxyethanol, thymol, sorbic acid, chlorobutanol, and others.

Examples of the paraben include methyl paraoxybenzoate, ethyl paraoxybenzoate, propyl paraoxybenzoate, and others.

The amount of the preservative in the pharmaceutical composition of the present invention may be suitably varied depending on, for example, the type of the preservative. The amount thereof is, for example, 0.001 to 1% (w/w). In addition, when the pharmaceutical composition of the present invention comprises a preservative, the preservatives may be used alone and two or more preservatives may be used in combination.

When the pharmaceutical composition of the present invention can produce the preservative efficacy required for pharmaceutical products without adding a preservative therein, it is preferable not to add a preservative, for example, a paraben in the pharmaceutical composition. For example, the criteria for evaluating the preservative efficacy required for pharmaceutical products is defined for each formulation category in the Japanese Pharmacopoeia 17th Edition. When each formulation meets the criteria, it is evaluated that the formulation has the preservative efficacy. Also, since epinastine or a salt thereof produces the preservative efficacy depending on the concentration thereof, the pharmaceutical composition of the present invention can achieve the desired preservative efficacy without the addition of a preservative, depending on the type of formulation. The desired preservative efficacy means, for example, that a formulation meets the criteria defined in the preservatives-effectiveness tests described in the Japanese Pharmacopoeia 17th Edition. Also, the irritation when the pharmaceutical composition of the present invention is administered transdermally can be reduced because no preservative is contained therein.

In the pharmaceutical composition of the present invention, a surfactant available as pharmaceutical additives may be added as appropriate. Examples of the surfactant include a cationic surfactant, an anionic surfactant, a non-ionic surfactant, and others.

Examples of the cationic surfactant include alkylamine salt, alkylamine-polyoxyethylene additive, fatty acid triethanolamine monoester salt, acylaminoethyldiethylamine salt, fatty acid-polyamine conjugate, alkyl imidazoline, 1-acylaminoethyl-2-alkyl imidazoline, 1-hydroxylethyl-2-alkyl imidazoline, and others.

Examples of the anionic surfactant include sodium alkylbenzene sulfonate, sodium dodecyl sulfate, a phospholipid such as lecithin, and others.

Examples of the non-ionic surfactant include polyoxyethylene fatty acid ester such as polyoxyl 40 stearate; sorbitan fatty acid ester such as sorbitan stearate; polyoxyethylene sorbitan fatty acid ester such as polysorbate 80; polyoxyethylene hydrogenated castor oil such as polyoxyethylene hydrogenated castor oil 60; polyoxyl castor oil such as polyoxyl 35 castor oil; polyoxyethylene polyoxypropylene glycol such as polyoxyethylene (20) polyoxypropylene (20); poloxyethylene polyoxypropylene cetyl ether such as polyoxyethylene (20) polyoxypropylene (4) cetyl ether; polyoxyethylene cetyl ether; polyoxyethylene lauryl ether; polyoxyethylene behenyl ether; glycerin fatty acid ester such as glyceryl laurate, polyglyceryl laurate, glyceryl palmitate, polyglyceryl palmitate, glyceryl stearate (glyceryl monostearate), polyglyceryl oleate, glyceryl linoleate, polyglyceryl linoleate, glyceryl linolenate, polyglyceryl linolenate, glyceryl ricinoleate, polyglyceryl ricinoleate, condensed ricinoleic acid polyglyceryl ester (also referred to as polyglyceryl polyricinoleate condensed ricinolenic acid polyglyceryl ester or polyglyceryl polyricinolenate); sucrose fatty acid ester such as sucrose stearate; and others.

The amount of the surfactant in the pharmaceutical composition of the present invention may be suitably varied depending on, for example, the type of the surfactant. The amount thereof is, for example, 0.1 to 20% (w/w), preferably 0.5 to 10% (w/w), more preferably 1 to 5% (w/w), but this is not applied if the surfactant produces any effect other than the effect as surfactant. In addition, when the pharmaceutical composition of the present invention comprises a surfactant, the surfactant may be used alone and two or more surfactants may be used in combination. Also, the surfactant may be used as a mixture of two or more surfactants.

The surfactant is characterized by the balance between the hydrophilic and lipophilic portions of molecules thereof and has a hydrophile-lipophile balance (HLB) value. The HLB value increases with increasing the hydrophilicity of a molecule and may vary by the manufacturer even with the same ingredient name.

The HLB value of the surfactant in the pharmaceutical composition of the present invention is not particularly limited as long as it is a value that can be prepared as a formulation for application to the skin. The HLB value is, for example, 1 to 20, preferably 1.0 to 10.0, more preferably 2.0 to 8.0, furthermore preferably 3.0 to 6.0.

In the pharmaceutical composition of the present invention, a cooling agent available as pharmaceutical additives may be added as appropriate. Examples of the cooling agent include terpenoid, essential oil containing terpenoid, and others.

Examples of the terpenoid include menthol, camphor, borneol, geraniol, nerol, cineole, citronellol, carvone, anethole, eugenol, limonene, linalool, linalyl acetate, and others. They may be in the form of d-, 1- or dl-body.

Examples of the essential oil containing terpenoid include eucalyptus oil, bergamot oil, peppermint oil, fennel oil, rose oil, cassia oil, spearmint oil, camphor oil, cool mint, mentha oil, and others.

The amount of the cooling agent in the pharmaceutical composition of the present invention may be suitably varied depending on, for example, the type of the cooling agent. The amount thereof is, for example, 0.001 to 1% (w/w). In addition, when the pharmaceutical composition of the present invention comprises a cooling agent, the cooling agent may be used alone and two or more cooling agents may be used in combination.

In the pharmaceutical composition of the present invention, an oil ingredient available as pharmaceutical additives may be added at appropriate. Examples of the oil ingredient include a hydrocarbon, a wax, an oil and fat, an aliphatic carboxylic acid or a salt thereof, a fatty acid ester, medium-chain triglyceride (MCT), a higher alcohol, and others.

Examples of the hydrocarbon include vaseline, white petrolatum, liquid paraffin (light liquid paraffin, heavy liquid paraffin), solid paraffin (paraffin), squalene, squalane, ceresin, microcrystalline wax, and others. They may be used as a mixture thereof. Examples of the mixture include mixture of paraffin and microcrystalline wax, and others.

Examples of the wax include beeswax, white beeswax, lanolin, and others.

Examples of the oil and fat include olive oil, castor oil, sesame oil, soybean oil, and others.

Examples of the aliphatic carboxylic acid include short-chain fatty acid such as butyric acid, valeric acid and caproic acid, medium-chain fatty acid such as caprylic acid and capric acid, long-chain fatty acid such as lauric acid, myristic acid, palmitic acid, stearic acid, behenic acid, oleic acid, linoleic acid, linolenic acid, ricinoleic acid (ricinolenic acid) and arachidonic acid, aliphatic dicarboxylic acid such as succinic acid, glutaric acid, adipic acid and sebacic acid, and others. It also includes branched aliphatic carboxylic acid. Examples of the salt of the aliphatic carboxylic acid include sodium salt thereof, and others.

The fatty acid ester as used herein refers to a compound generated by the ester binding of the carboxyl group of an aliphatic carboxylic acid with an alcohol. Examples of the alcohol that may be esterified to the carboxyl group of an aliphatic carboxylic acid include a monovalent alcohol such as methanol, ethanol, n-propanol, n-butanol, n-pentanol, n-hexanol, n-heptanol, n-octanol, isopropanol, isobutanol, sec-butanol and isopentanol, a polyhydric alcohol such as ethylene glycol, diethylene glycol, propylene glycol, butylene glycol and glycerin, a multimeric complex (polymer) of polyhydric alcohol such as dipolyethylene glycol, polyethylene glycol, dipropylene glycol, polypropylene glycol, diglycerin, triglycerin and polyglycerin, and others. Specific examples thereof include polyethylene glycol laurate, isopropyl myristate, octyl myristate, glycol palmitate, polyethylene glycol stearate, isopropyl oleate, propylene glycol linoleate, ethyl linolenate, ethylene glycol ricinoleate (ricinolenate), diisopropyl adipate, and others. When the fatty acid ester has two or more ester bonds, the alcohols for forming the ester bonds may be the same or different.

Examples of the higher alcohol include lauryl alcohol, myristyl alcohol, palmityl alcohol (cetyl alcohol), stearyl alcohol, behenyl alcohol, oleyl alcohol, linoleyl alcohol, linolenyl alcohol, octydodecanol, and others.

The amount of the oil ingredient in the pharmaceutical composition of the present invention may be suitably varied depending on, for example, the type of the oil ingredient. The amount thereof is, for example, 0.1 to 50% (w/w), preferably 1 to 40% (w/w), more preferably 10 to 30% (w/w). When the dosage form of the pharmaceutical composition of the present invention is ointment formulation, the amount thereof may be, for example, 50% (w/w) or more. In addition, when the pharmaceutical composition of the present invention comprises an oil ingredient, the oil ingredient may be used alone and two or more oil ingredients may be used in combination.

When the oil ingredient in the pharmaceutical composition of the present invention acts as surfactant, the oil ingredient may be read as surfactant.

The oil ingredient in the pharmaceutical composition of the present invention may be used as a solubilizing agent (solubilizer) for improving the solubility of other ingredients contained therein. The oil ingredient in the pharmaceutical composition of the present invention may be used as a solubilizing agent for improving the solubility of epinastine or a salt thereof and a sulfur-based antioxidant.

The pharmaceutical composition of the present invention may further comprise a solvent and/or a dispersion medium. The pharmaceutical composition of the present invention comprising a solvent and/or a dispersion medium may be in the state that all of the ingredients contained therein are dissolved or a portion thereof is suspended, or may be in the form of emulsion or semi-solid. Examples of the solvent and/or the dispersion medium include water, ethanol, polyol (e.g., glycerin (glycerol), propylene glycol, 1,3- butylene glycol, liquid polyethylene glycol, macrogol), and others, but is not limited thereto.

The amount of the solvent and/or the dispersion medium in the pharmaceutical composition of the present invention may be suitably varied depending on, for example, the type of the solvent and the dispersion. For example, the amount thereof is preferably 10% (w/w) or more, more preferably 30% (w/w) or more, relative to the total amount of the pharmaceutical composition. In addition, when the pharmaceutical composition of the present invention comprises a solvent and/or a dispersion medium, the solvent and/or the dispersion medium may be used alone and two or more solvents and/or dispersion media may be used in combination.

When the pharmaceutical composition of the present invention is used as an emulsion, the pharmaceutical composition may be in the form of oil-in-water emulsion (emulsion consisting of oil droplets dispersed in water phase which is the continuous phase) or water-in-oil emulsion (emulsion consisting of aqueous droplets dispersed in oil phase which is the continuous phase). The pharmaceutical composition of the present invention is preferably water-in-oil emulsion.

The average size of the oil or aqueous droplets is, for example, 20 to 3000 nm, preferably 50 to 2000 nm, more preferably 100 to 1000 nm, furthermore preferably 200 to 800 nm.

The pharmaceutical composition of the present invention can be prepared according to a usual method commonly used in the art. For example, the pharmaceutical composition can be prepared by mixing an active ingredient and a sulfur-based antioxidant with an additive such as a stabilizing agent, a preservative, a surfactant, an oil ingredient and a solvent or dispersion medium such as water. Also, the pharmaceutical composition can be prepared as a sterile formulation according to a usual sterilization method commonly used in the art as appropriate. The sterilization method is not particularly limited as long as it is a method which can be used in the preparation process. Examples thereof include high-pressure steam sterilization, filtration sterilization, dry heat sterilization, electron beam (EB) sterilization, γ-ray sterilization, ethylene oxide gas (EOG) sterilization and hydrogen peroxide gas sterilization.

The pharmaceutical composition of the present invention may comprise a pharmaceutical active ingredient other than epinastine or a salt thereof, unless otherwise noted. When the pharmaceutical composition of the present invention is, for example, an ophthalmic pharmaceutical composition, the pharmaceutical composition may comprise a pharmaceutical active ingredient used in ophthalmic solution other than epinastine or a salt thereof. Examples of other pharmaceutical active ingredient include an antiinflammatory agent, an anti-bacterial agent, an anti-viral agent, a vitamin agent, a vasoconstrictive agent, a mydriatic agent, a miotic agent, an ocular hypotensive agent, an agent for treating dry eye, a local anesthetic agent, and others. Also, the pharmaceutical composition of the present invention may comprise epinastine or a salt thereof as the only active ingredient.

When the pharmaceutical composition of the present invention is used as an ocular pharmaceutical composition, the pharmaceutical composition is particularly useful as an agent for treating allergic conjunctivitis. The term "treatment of allergic conjunctivitis" as used herein refers to every treatment (e.g., improvement, alleviation, inhibition of progression) of allergic conjunctivitis and the associated symptoms and prophylaxis thereof.

When the pharmaceutical composition of the present invention is used as an otorhinological pharmaceutical composition, the pharmaceutical composition is particularly useful as an agent for treating allergic rhinitis. The term "treatment of allergic rhinitis" as used herein refers to every treatment (e.g., improvement, alleviation, inhibition of progression) of allergic rhinitis and the associated symptoms and prophylaxis thereof.

When the pharmaceutical composition of the present invention is used as a dermatological pharmaceutical composition, the pharmaceutical composition is particularly useful as an agent for treating allergic dermatitis such as contact dermatitis, eczema dermatitis, atopic dermatitis and urticaria. The term "treatment of allergic dermatitis" as used herein refers to every treatment (e.g., improvement, alleviation, inhibition of progression) of allergic dermatitis and the associated symptoms and prophylaxis thereof.

The term "patient" as used herein refers to human and an animal such as dog, cat, and house. The patient is preferably a mammal, more preferably human. The term "therapeutically effective amount" as used herein refers to an amount for producing the therapeutic effect on a disease or the associated symptoms or an amount for delaying the onset or progression of a disease or the associated symptoms, relative to untreated subjects.

The dose and usage of the pharmaceutical composition of the present invention are not particularly limited as long as they are sufficient to produce the desired efficacy. The pharmaceutical composition may be administrated once to four times daily, preferably once to twice daily, more preferably once daily.

When the pharmaceutical composition of the present invention is used as an ointment formulation, cream formulation or gel formulation, the dose thereof is not particularly limited as long as it is sufficient to produce the desired efficacy. Also, the dose varies depending on the amount of active ingredient and the patient. For example, a cream formulation may be administered transdermally at an appropriate amount, specifically about 1 mg to about 5 g, preferably about 5 mg to about 1 g, more preferably about 10 mg to about 500 mg, particularly preferably about 20 mg to about 100 mg, per time in an adult. For example, the dose thereof is 30 mg. As an example of the administration, a patient himself may take an appropriate amount, for example, about 20 to 40 mg of the pharmaceutical composition of the present invention prepared as an ophthalmic pharmaceutical composition on his finger and apply it to the upper and lower eyelid skin of one eye in about half the amount and then may apply it to the other eye in the same manner. In actual use, the patient may use the pharmaceutical composition in a rough amount without weighing it, using the aforementioned dose for each eye as a standard amount. In addition, the dose varies depending on the size and shape of the container filled with the pharmaceutical composition of the present invention, but the pharmaceutical composition may be used in an amount of 0.5 FTU or 1 FTU when the amount of the pharmaceutical composition loaded from the tip of an adult index finger to the first joint is defined as 1 FTU (1 Finger Tip Unit) as a standard amount.

The application time when applied to the skin is preferably 0.5 to 24 hours, more preferably 2 to 12 hours, furthermore preferably 4 to 8 hours. If the pharmaceutical composition is removed from the skin after the application time, a sufficient amount of the active ingredient therein is retained in the skin tissue. As a result, it is expected that the active ingredient is released slowly, resulting in the sustained efficacy. One example of the usage is to apply the pharmaceutical composition of the present invention to the eyelid skin before bedtime and remove it from the skin after waking up. Thereby, it is expected that the effect of treating and preventing allergic conjunctivitis is sustained without application to the skin during the daytime.

When the pharmaceutical composition of the present invention is used as ointment formulation, cream formulation or gel formulation, the container for the pharmaceutical composition is not particularly limited, but the pharmaceutical composition may be contained in a tube, a bottle, a can or any other container. The material of container is not particularly limited, but the container may be a resin container made of a material such as polyethylene (PE), polypropylene (PP), or polyethylene terephthalate (PET), polybuthylene terephthalate (PBT), polypropylene and polyethylene copolymer, polyvinyl chloride, acrylic resin, polystyrene, polycyclic olefin copolymer, a metal container made of a material such as aluminum, or a laminate container produced by laminating several materials such as resin, paper, aluminum foil. For example, when the material of a resin container is polyethylene, a container made of low-density polyethylene (LDPE), liner low-density polyethylene (LLDPE), medium-density polyethylene (MDPE) or high-density polyethylene (HDPE), which is polyethylene classified by its density, may be used.

The pharmaceutical composition of the present invention may be used both when wearing hard contact lenses and when wearing soft contact lenses.

The pharmaceutical composition of the present invention can inhibit the generation and increase of the related substance(s) of epinastine or a salt thereof. The "related substance" as used herein refers to an impurity of the drug substance of epinastine or a salt thereof and a degradation product of epinastine or a salt thereof.

### EXAMPLES

Hereinafter, the present invention is illustrated in Formulation Examples and the results of each test in order to make it easy to understand the present invention. The present invention, however, is not intended to be limited thereto by any means.

### Formulation Examples

Typical examples of the formulation of the present invention are shown below. The amount of each ingredient formulated in the following formulation examples is the amount in 100 g of the formulation. Also, the term "% (w/w)" refers to the amount (g) of each ingredient in 100 g of the formulation.

| [Formulation Example 1] Water-in-oil emulsion | |
|---|---|
| Epinastine | 1.0 g |
| Squalane | 5.0 g |
| Light Liquid Paraffin | 5.0 g |
| Ceresin | 3.0 g |
| White Petrolatum | 10.0 g |
| White Beeswax | 1.0 g |
| Glycerin Fatty Acid Ester | 5.0 g |
| Propyl Paraoxylbenzoate | 0.1 g |
| Methyl Paraoxylbenzoate | 0.1 g |
| 2-Mercaptobenzimidazole | 0.1 g |
| Sodium Edetate Hydrate | 0.5 g |
| Concentrated Glycerin | 15.0 g |
| Purified Water | q.s. |

| [Formulation Example 2] Water-in-oil emulsion | |
|---|---|
| Epinastine Hydrochloride | 2.0 g |
| Cetyl Alcohol | 0.5 g |
| White Petrolatum | 8.0 g |
| Isopropyl Myristate | 4.0 g |
| Polysorbate 80 | 0.5 g |
| Propyl Paraoxylbenzoate | 0.05 g |
| Methyl Paraoxylbenzoate | 0.03 g |
| Dibutylhydroxytoluene | 0.2 g |
| 2-Mercaptobenzimidazole | 0.05 g |
| Propylene Glycol | 5.0 g |
| Sodium Hydroxide | q.s. |
| Purified Water | q.s. |

| [Formulation Example 3] Water-in-oil emulsion | |
|---|---|
| Epinastine Hydrochloride | 3.0 g |
| Cetyl Alcohol | 2.0 g |
| Squalane | 10.0 g |
| Glycerin Fatty Acid Ester | 4.0 g |
| Polyoxyethylene Hydrogenated Castor Oil | 0.5 g |
| 2-Mercaptobenzimidazole | 0.3 g |
| Octyldodecanol | 5.0 g |
| Sodium Edetate Hydrate | 0.5 g |
| Concentrated Glycerin | 10.0 g |
| Sodium Hydroxide | q.s. |
| Purified Water | q.s. |

| [Formulation Example 4] Oil-in-water emulsion | |
|---|---|
| Epinastine Hydrochloride | 1.0 g |
| Squalane | 10.0 g |
| Glyceryl Monostearate | 4.0 g |
| Polyoxyethylene Behenyl Ether | 4.0 g |
| Polyoxyethylene Hydrogenated Castor Oil | 1.0 g |
| Cetyl Alcohol | 5.0 g |
| Stearyl Alcohol | 2.5 g |
| 2-Mercaptobenzimidazole | 0.1 g |
| Diisopropyl Adipate | 4.0 g |
| 1,3-Butylene Glycol | 5.0 g |
| Sodium Citrate | q.s. |
| Sodium Hydroxide | q.s. |
| Purified Water | q.s. |

In addition, formulation examples 5 to 36 are shown in Tables 1 to 4.

**[Table 1]**

| Ingredient [%(w/w)] | Formulation Example | | | | | | | |
|---|---|---|---|---|---|---|---|---|
| | 5 | 6 | 7 | 8 | 9 | 10 | 11 | 12 |
| Epinastine Hydrochloride | 0.05 | 0.05 | 0.05 | 0.05 | 0.05 | 0.05 | 0.05 | 0.05 |
| White Petrolatum | 10 | 5.0 | 5.0 | 8.0 | 5.0 | 8.0 | 8.0 | 5.0 |
| Light Liquid Paraffin | 8.0 | 10 | 5.0 | 8.0 | 8.0 | 8.0 | 5.0 | 5.0 |
| Squalane | 8.0 | 5.0 | 10 | 8.0 | 8.0 | 5.0 | 4.0 | 5.0 |
| Mixture of Paraffin and Microcrystalline Wax | 4.0 | 4.0 | 4.5 | 2.5 | 3.0 | 5.0 | 4.5 | 3.0 |
| White Beeswax | 1.0 | 1.0 | 0.5 | 1.5 | 1.0 | 0.5 | 1.5 | 1.5 |
| Glycerin Fatty Acid Ester | 5.5 | 4.5 | 4.5 | 4.5 | 4.0 | 4.0 | 4.0 | 5.0 |
| 2-Mercaptobenzimidazole | 0.1 | 0.05 | 0.05 | 0.05 | 0.05 | 0.1 | 0.1 | 0.05 |
| Sodium Edetate Hydrate | 0.05 | 0.75 | 0.75 | 0.05 | 0.03 | 0.75 | 0.05 | 0.1 |
| Concentrated Glycerin | 8.0 | 10 | 11 | 12 | 13 | 14 | 14 | 15 |
| Sodium Chloride | 0.5 | 0.5 | 0.45 | 0.4 | 0.35 | 0.3 | 0.25 | 0.5 |
| Sodium Hydroxide | q.s. | | | | | | | |
| Purified Water | q.s. | | | | | | | |

**[Table 2]**

| Ingredient [%(w/w)] | Formulation Example | | | | | | | |
|---|---|---|---|---|---|---|---|---|
| | 13 | 14 | 15 | 16 | 17 | 18 | 19 | 20 |
| Epinastine Hydrochloride | 0.5 | 0.5 | 0.5 | 0.5 | 0.5 | 0.5 | 0.5 | 0.5 |
| White Petrolatum | 10 | 5.0 | 5.0 | - | - | 8.0 | 10 | 15 |
| Light Liquid Paraffin | - | 10 | 5.0 | 5.0 | 5.0 | 8.0 | 8.0 | - |
| Squalane | 10 | - | 8.0 | 5.0 | 5.0 | - | 5.0 | 2.0 |
| Lanolin | - | - | - | 15 | 10 | 5.0 | - | - |
| Mixture of Paraffin and Microcrystalline Wax | 1.0 | 1.0 | 1.0 | 1.0 | 1.0 | 1.0 | 1.0 | 1.0 |
| White Beeswax | - | - | - | 1.0 | - | - | - | 5.0 |
| Glycerin Fatty Acid Ester | - | 5.0 | - | - | - | - | - | 5.0 |
| Polyethylene Glycol Stearate | 8.0 | - | - | - | - | 6.0 | 4.0 | |
| Glyceryl Monooleate | - | - | 8.0 | 4.0 | 4.0 | - | - | - |
| 2-Mercaptobenzimidazole | 0.05 | 0.05 | 0.05 | 1.0 | 1.0 | 1.0 | 1.0 | 1.0 |
| Octyldodecanol | 5.0 | 5.0 | - | - | - | 8.0 | 8.0 | 3.0 |
| Cetyl Alcohol | - | - | 7.5 | 7.5 | - | - | - | 3.0 |
| Sodium Edetate Hydrate | - | 0.05 | 0.05 | 0.05 | 0.05 | 0.05 . | - | - |
| Concentrated Glycerin | 8.0 | 10 | - | 12 | - | 15 | - | - |
| Propylene Glycol | - | - | 10 | - | 12 | - | 15 | 15 |
| Sodium Chloride | 0.5 | 0.5 | 1.0 | 0.3 | 1.0 | - | - | 0.8 |
| Sodium Hydroxide | q.s. | | | | | | | |
| Purified Water | q.s. | | | | | | | |

**[Table 3]**

| Ingredient [%(w/w)] | Formulation Example | | | | | | | |
|---|---|---|---|---|---|---|---|---|
| | 21 | 22 | 23 | 24 | 25 | 26 | 27 | 28 |
| Epinastine Hydrochloride | 1.0 | 1.0 | 1.0 | 1.0 | 0.5 | 0.5 | 0.5 | 0.5 |
| White Petrolatum | 10 | 15 | 5.0 | 10 | 5.0 | 10 | 8.0 | 8.0 |
| Light Liquid Paraffin | 5.0 | - | 10 | - | 5.0 | 5.0 | 8.0 | 5.0 |
| Squalane | 8.0 | 5.0 | 5.0 | 10 | 5.0 | - | 8.0 | 5.0 |
| Ceresin | 1.0 | 1.0 | - | 1.0 | - | - | 1.0 | 2.0 |
| Glycerin Fatty Acid Ester | - | - | 5.0 | - | - | 2.0 | 4.0 | - |
| Polyoxyethylene Hydrogenated Castor Oil 60 | 5.0 | - | - | 5.0 | - | - | 3.0 | - |
| Polysorbate 80 | - | 5.0 | - | - | 5.0 | - | - | - |
| Medium-Chain Triglyceride | - | - | - | 10 | 10 | 15 | - | 10 |
| 2-Mercaptobenzimidazole | 0.05 | 0.05 | 0.05 | 0.1 | 0.1 | 0.05 | 0.05 | 0.05 |
| Polyoxyethylene Cetyl Ether | - | - | 3.0 | 3.0 | 3.0 | 3.0 | - | - |
| Cetyl Alcohol | 8.0 | 8.0 | 8.0 | 8.0 | 8.0 | - | - | - |
| Stearyl Alcohol | - | - | - | - | - | 5.0 | 5.0 | 5.0 |
| Sodium Edetate Hydrate | 0.03 | 0.03 | 0.05 | 0.05 | 0.03 | 0.03 | 0.05 | 0.05 |
| Concentrated Glycerin | 15 | 15 | - | - | - | - | 12 | 12 |
| Macrogol 400 | - | - | 20 | 20 | 15 | 15 | - | - |
| Sodium Citrate Hydrate | 1.0 | 1.0 | 0.5 | 0.5 | 1.0 | 1.0 | 0.5 | 0.5 |
| Sodium Hydroxide | q.s. | | | | | | | |
| Purified Water | q. s. | | | | | | | |

**[Table 4]**

| Ingredient [%(w/w)] | Formulation Example | | | | | | | |
|---|---|---|---|---|---|---|---|---|
| | 29 | 30 | 31 | 32 | 33 | 34 | 35 | 36 |
| Epinastine Hydrochloride | 0.5 | 0.5 | 0.5 | 0.5 | 0.5 | 0.5 | 0.5 | 0.5 |
| White Petrolatum | 10 | 5.0 | 5.0 | 8.0 | 5.0 | 8.0 | 8.0 | 5.0 |
| Light Liquid Paraffin | 8.0 | 10 | 5.0 | 8.0 | 8.0 | 8.0 | 5.0 | 5.0 |
| Squalane | 8.0 | 5.0 | 10 | 8.0 | 8.0 | 5.0 | 4.0 | 5.0 |
| Mixture of Paraffin and Microcrystalline Wax | 3.0 | 4.0 | 4.5 | 2.5 | 3.0 | 5.0 | 4.5 | 4.0 |
| White Beeswax | 1.5 | 1.0 | 0.5 | 1.5 | 1.0 | 0.5 | 1.5 | 1.0 |
| Glycerin Fatty Acid Ester | 5.0 | 4.5 | 4.5 | 4.5 | 4.0 | 4.0 | 4.0 | 5.5 |
| 2-Mercaptobenzimidazole | 0.05 | 0.05 | 0.05 | 0.05 | 0.05 | 0.1 | 0.1 | 0.1 |
| Sodium Edetate Hydrate | 0.1 | 0.75 | 0.75 | 0.05 | 0.03 | 0.75 | 0.05 | 0.05 |
| Concentrated Glycerin | 8.0 | 10 | 11 | 12 | 13 | 14 | 14 | 15 |
| Sodium Chloride | 0.5 | 0.5 | 0.45 | 0.4 | 0.35 | 0.3 | 0.25 | 0.25 |
| Sodium Hydroxide | q.s. | | | | | | | |
| Purified Water | q.s. | | | | | | | |

### Examples

### 1. Stability test of epinastine or salt thereof (1)

### (1) Test procedure

According to a commonly-used method, epinastine hydrochloride, which is an active ingredient, an oil base, each antioxidant, sodium hydroxide and purified water were mixed to prepare test formulations 1 to 4. Also, test formulation 5 was prepared according to a similar method to the method of preparing test formulations 1 to 4 except that no antioxidant is added. The prepared test formulations 1 to 5 were stored in a storage room at a temperature of 60°C for 1 week, respectively. The amounts of related substances immediately after each formulation was prepared and after each formulation was stored for 1 week were quantified according to the following analysis condition, and the quantified amounts were compared.

### <Analysis condition>

Column: Stainless steel tube with an inner diameter of 4.6 mm and a length of 15 cm filled with 5 µm of octadecylsilyl silica gel for liquid chromatography
Mobile phase: Concentration gradient by Mobile phase A (aqueous solution comprising potassium dihydrogen phosphate, sodium 1-pentanesulfonate, methanol, phosphate buffer) and Mobile phase B (acetonitrile-methanol mixed solution)
Flow rate: 0.7 mL/min
Detection device: UV absorption spectrophotometer
(Measurement wavelength: 220 nm)
Column temperature: Constant temperature around 50°C

The ingredients added in test formulations 1 to 5 are described in Table 5. The "oil base" in Table 5 comprises each of squalane, stearyl alcohol, cetyl alcohol, glyceryl monostearate, polyoxyethylene hydrogenated castor oil 100, polyoxyethylene behenyl ether 20, polyoxyethylene (20) polyoxypropylene (4) cetyl ether, diisopropyl adipate, and propyl paraoxylbenzoate in appropriate amounts, and the ratio of each ingredient in the oil base is the same for test formulations 1 to 5. Also, sodium hydroxide and purified water are contained in appropriate amounts for the dissolution and neutralization of epinastine hydrochloride.

**[Table 5]**

| Ingredient [%(w/w)] | | Test Formulation | | | | |
|---|---|---|---|---|---|---|
| | | 1 | 2 | 3 | 4 | 5 |
| Epinastine Hydrochloride | | 2.6 | | | | 2.6 |
| Oil Base | | 80.1 | | | | 80.5 |
| Antioxidant | BHT | 0.5 | - | - | - | - |
| | Tocopherol Acetate | - | 0.5 | - | - | - |
| | Propyl Gallate | - | - | 0.5 | - | - |
| | MBI | - | - | - | 0.5 | - |
| Sodium Hydroxide and Purified Water | | 16.8 | | | | 16.9 |

### (2) Test results and discussion

The amounts of main related substances for each test formulation are shown in Table 6. As for the amounts of the related substances after the formulations were prepared and stored for 1 week, it was found that the amount of the related substance with a relative retention time (RRT) of 0.142 increased in not only test formulation 5 without any antioxidant but also test formulations 1 to 3 comprising each antioxidant. On the other hand, it was found that the increase in the amount of the related substance was inhibited in test formulation 4 comprising 2-mercaptobenzimidazole as an antioxidant. In addition, it was found that the amount of the related substance with a RRT of 1.035 increased in test formulation 5 without any antioxidant, test formulation 2 comprising tocopherol acetate as an antioxidant and test formulation 3 comprising propyl gallate as an antioxidant, whereas the increase in the amount of the related substance was inhibited in test formulation 1 comprising dibutylhydroxytoluene and test formulation 4 comprising 2-mercaptobenzimidazole.

**[Table 6]**

| Related Substance [%] | Measured Time Point | Test Formulation | | | | |
|---|---|---|---|---|---|---|
| | | 1 | 2 | 3 | 4 | 5 |
| RRT 0.142 | Immediately after preparation | 0.16 | 0.27 | 0.40 | 0.10 | 0.24 |
| | After 1 week | 0.25 | 0.42 | 0.92 | 0.11 | 0.54 |
| RRT 1.035 | Immediately after preparation | 0.05 | 1.15 | 0.13 | 0.01 | 0.06 |
| | After 1 week | 0.05 | 7.33 | 0.31 | 0.07 | 0.17 |

### 2. Stability test of epinastine or salt thereof (2)

### (1) Test procedure

According to a commonly-used method, epinastine hydrochloride, which is an active ingredient, a base, each antioxidant, sodium hydroxide, sodium chloride and purified water were mixed to prepare test formulations 6 to 9 in the form of water-in-oil emulsion. Also, test formulation 10 in the form of water-in-oil emulsion was prepared according to a similar method to the method of preparing test formulations 6 to 9 except that no antioxidant is added. The prepared test formulations 6 to 10 were stored in a storage room at a temperature of 40°C and a humidity of 75% for 1 month, respectively. The amounts of related substances immediately after each formulation was prepared and after each formulation was stored for 1 week were quantified according to the similar analysis condition to the above "1. Stability test of epinastine or salt thereof (1)", and the quantified amounts were compared.

The ingredients added in test formulations 6 to 10 are described in Table 7. The "base" in Table 7 comprises each of squalane, white petrolatum, light liquid paraffin, ceresin, white beeswax, concentrated glycerin, octyldodecanol, glycerin fatty acid ester, methyl paraoxylbenzoate, propyl paraoxylbenzoate, and sodium edetate hydrate in appropriate amounts, and the ratio of each ingredient in the base is the same for test formulations 6 to 10.

**[Table 7]**

| Ingredient [%(w/w)] | | Test Formulation | | | | |
|---|---|---|---|---|---|---|
| | | 6 | 7 | 8 | 9 | 10 |
| Epinastine Hydrochloride | | 0.5 | | | | |
| Base | | 42.2 | | | | |
| Antioxidant | BHT | 0.1 | 0.2 | - | 0.2 | - |
| | MBI | - | - | 0.05 | 0.05 | - |
| Sodium Hydroxide, Sodium Chloride and Purified Water | | q.s. | | | | |

### (2) Test results and discussion

The amounts of the total related substances and the related substance with a RRT of 1.064 for each test formulation are shown in Table 8. As for the amounts of the related substances after formulations were prepared and stored for 1 month, it was found that the amount of the related substance with a RRT of 1.064 increased in test formulation 10 without any antioxidant, and the amount thereof tended to increase regardless of the concentration of dibutylhydroxytoluene in test formulations 6 and 7 comprising dibutylhydroxytoluene as an antioxidant. On the other hand, it was found that the increase in the amount of the related substance was inhibited in test formulations 8 and 9 comprising 2-mercaptobenzimidazole. Also, it was found that test formulations 8 and 9 could inhibit the increase in the amount of the total related substances.

**[Table 8]**

| Related Substance [%] | | Test Formulation | | | | |
|---|---|---|---|---|---|---|
| | | 6 | 7 | 8 | 9 | 10 |
| Total Related Substance | Immediately after preparation | 0.17 | 0.14 | 0.19 | 0.11 | 0.24 |
| | After 1 month | 0.55 | 0.54 | 0.45 | 0.40 | 0.74 |
| RRT 1.064 | Immediately after preparation | 0.10 | 0.08 | 0.05 | 0.04 | 0.17 |
| | After 1 month | 0.27 | 0.25 | 0.10 | 0.09 | 0.42 |

### 3. Long-term stability test

### (1) Test procedure

Test formulations 8 and 10 described in the above "2. Stability test of epinastine or salt thereof (2)" were prepared and the prepared formulations were stored in a storage room at a temperature of 40°C and a humidity of 75% for 6 months. The amounts of the related substances immediately after each formulation was prepared and after each formulation was stored for 6 months were quantified according to the similar analysis condition to the above "1. Stability test of epinastine or salt thereof (1)", and the quantified amounts were compared.

### (2) Test results and discussion

The amounts of the total related substances and the related substance with a RRT of 1.05 for each formulation are shown in Table 9. As for the amounts of the related substances after formulations were prepared and stored for 6 months, it was found that the amount of the related substance with a RRT of 1.05 increased in test formulation 10 without any antioxidant, whereas the increase in the amount thereof was inhibited in test formulation 8 comprising 2-mercaptobenzimidazole. Also, it was found that test formulation 8 could inhibit the increase in the amount of the total related substances as compared to test formulation 10.

**[Table 9]**

| Related Substance [%] | | Test Formulation | |
|---|---|---|---|
| | | 8 | 10 |
| Total Related Substance | Immediately after preparation | 0.10 | 0.30 |
| | After 6 months | 0.87 | 1.67 |
| RRT 1.05 | Immediately after preparation | < 0.05 | 0.12 |
| | After 6 months | 0.17 | 0.49 |

### 4. Stability test of epinastine or salt thereof (3)

### (1) Test procedure

According to a commonly-used method, epinastine hydrochloride, which is an active ingredient, a base, each antioxidant, sodium hydroxide, sodium chloride and purified water were mixed to prepare test formulations 11 to 15 in the form of water-in-oil emulsion. The prepared test formulations 11 to 15 were stored in a storage room at a temperature of 60°C for 4 weeks, respectively. The amounts of the related substances immediately after each formulation was prepared and after each formulation was stored for 4 weeks were quantified according to the similar analysis condition to the above "1. Stability test of epinastine or salt thereof (1)", and the quantified amounts were compared.

The ingredients added in test formulations 11 to 14 are described in Table 10. Also, the ingredients added in test formulation 15 are described in Table 11 with reference to the aforementioned Patent Document 2 (WO 2007/007851).

**[Table 10]**

| Ingredient [%(w/w)] | | Test Formulation | | | |
|---|---|---|---|---|---|
| | | 11 | 12 | 13 | 14 |
| Epinastine Hydrochloride | | 0.5 | | | |
| Base | Light Liquid Paraffin | 8.0 | | | |
| | Squalane | 5.0 | | | |
| | White Petrolatum | 5.0 | | | |
| | Paraffin and Microcrystalline Wax Mixture | 3.0 | | | |
| | White Beeswax | 1.0 | | | |
| | Glycerin Fatty Acid Ester | 4.0 | | | |
| | Octyldodecanol | 3.0 | | | |
| | Concentrated Glycerin | 13.0 | | | |
| Antioxidant | MBI | 0.2 | - | - | - |
| | L-Cysteine Hydrochloride Hydrate | - | 0.2 | - | - |
| | L-Methionine | - | - | 0.2 | - |
| | Tocopherol (Vitamin E) | - | - | - | 0.2 |
| Sodium Hydroxide | | q.s. | | | |
| Sodium Chloride | | q.s. | | | |
| Purified Water | | q.s. | | | |

**[Table 11]**

| Ingredient [%(w/w)] | | Test Formulation |
|---|---|---|
| | | 15 |
| Epinastine Hydrochloride | | 0.5 |
| Base | White Petrolatum | 10.0 |
| | Cetyl Alcohol | 3.0 |
| | Stearic Acid | 2.0 |
| | Isopropyl Myristate | 40 |
| | Polysorbate 80 | 5.0 |
| | Propylene Glycol | 5.0 |
| | Methylparaben | 0.2 |
| | Propylparaben | 0.02 |
| Antioxidant | Ascorbic Acid (Vitamin C) | 1.0 |
| Purified Water | | q.s. |

### (2) Test results and discussion

The amounts of the total related substances for each test formulation are shown in Table 12. According to the results, it was found that the amount of the total related substances after 4 weeks of the storage greatly increased in test formulation 14 comprising tocopherol (Vitamin E) as an antioxidant and test formulation 15 comprising ascorbic acid (Vitamin C) as an antioxidant, whereas test formulations 11 to 13 comprising a sulfur-based antioxidant tended to inhibit the increase in the amount of the total related substances after 4 weeks of the storage.

**[Table 12]**

| Total related substance [%] | Test Formulation | | | | |
|---|---|---|---|---|---|
| | 11 | 12 | 13 | 14 | 15 |
| Immediately after preparation | 0 | 0.08 | 0.07 | 2.29 | 2.20 |
| After 4 weeks | 2.28 | 1.35 | 2.05 | 19.15 | 6.47 |

### INDUSTRIAL APPLICABILITY

The present invention provides a pharmaceutical composition for transdermal administration comprising epinastine or a salt thereof as an active ingredient which can maintain the effective concentration of epinastine or a salt thereof in an ocular tissue and stabilize epinastine or a salt thereof in the pharmaceutical composition for a long time.

## Claims

1. A pharmaceutical composition for transdermal administration comprising epinastine or a salt thereof and a sulfur-based antioxidant.

2. The pharmaceutical composition according to claim 1, wherein the sulfur-based antioxidant is one or more selected from the group consisting of cysteine, N-acetylcysteine, methionine, glutathione, 2-mercaptobenzimidazole, sodium thiomalate, thioglycerol, sodium thioglycolate, sodium thiosulfate, sodium hydrogen sulfite and sodium sulfite.

3. The pharmaceutical composition according to claim 2, wherein the sulfur-based antioxidant is 2-mercaptobenzimidazole.

4. The pharmaceutical composition according to any one of claims 1 to 3, wherein the concentration of the epinastine or a salt thereof is 0.01 to 50 (w/w).

5. The pharmaceutical composition according to any one of claims 1 to 4, wherein the pharmaceutical composition is ointment formulation, cream formulation or gel formulation.

6. The pharmaceutical composition according to claim 5, wherein the pharmaceutical composition is cream formulation.

7. The pharmaceutical composition according to any one of claims 1 to 6, wherein the pharmaceutical composition is water-in-oil emulsion.

8. The pharmaceutical composition according to any one of claims 1 to 7 for ophthalmic use.

9. The pharmaceutical composition according to any one of claims 1 to 8, wherein the transdermal administration is the administration to eyelid skin.

10. The pharmaceutical composition according to any one of claims 1 to 9 for treating allergic conjunctivitis.

11. The pharmaceutical composition according to any one of claims 1 to 10 for use in the administration to a patient once daily.

12. A pharmaceutical composition for transdermal administration comprising epinastine or a salt thereof and a sulfur-based antioxidant, wherein the pharmaceutical composition further comprises one or more oil ingredients selected from the group consisting of a hydrocarbon, a wax, an oil and fat, an aliphatic carboxylic acid or a salt thereof, a fatty acid ester and a higher alcohol.

13. The pharmaceutical composition according to claim 12 which further comprises a solvent and/or a dispersion medium.

14. The pharmaceutical composition according to claim 12 or 13, wherein the pharmaceutical composition is free of a paraben.
